# EUROPEAN PATENT APPLICATION

(11) **EP 4 450 066 A1**
(43) Date of publication of application: **23.10.2024**
(21) Application number: 23169291.4
(22) Date of filing: 21.04.2023
(51) Int. Cl.: A61K 9/16, A61K 9/20, A61K 35/76

(54) **PHAGE COMPOSITION**

(71) Applicant: Université Libre de Bruxelles, 1050 Bruxelles (BE)
(72) Inventor: GOOLE, Jonathan, 1950 Kraainem (BE); TABARE, Emilie, 1050 Ixelles (BE)
(74) Representative: Calysta NV

(57) **Abstract**

A process for the production of a dry composition comprising a phage comprising the steps of: selecting a phage and formulating it in an aqueous solution, preparing an aqueous solution comprising a colonic/enteric-release polymeric matrix agent, preparing an aqueous solution comprising trehalose and an amino acid selected from the group consisting of valine, leucine, isoleucine, phenylalanine and mixtures thereof and of submitting these solutions to a spray drying step, wherein the temperature of the drying gas at the inlet is lower than 100°C, and the corresponding edible pharmaceutical composition.

## Description

### Technical Field

The present invention relates to phage microparticles for oral administration, the production process and their use.

### Prior art

Antibiotic-resistant bacteria are increasing worldwide. The use of lytic bacteriophages is one of the interesting alternatives to treat bacterial infections.

However stable phage formulations are not easy to achieve, especially the drying step risks to affect phage viability.

Among the various administration routes, oral delivery is usually the most convenient, especially for an enteric delivery, and most likely to be accepted by patients. Oral dosage forms may be interestingly used to treat local pathology of the gastrointestinal tract, as well as infections of the urinary tract. However, oral administration of compounds, especially of biological compounds risks to markedly affect the stability of the administered compound in view of the high stomachal acidity.

Other administration modes do exist, yet correct drying and protection (during the drying step and after the administration to the patient) of the active substance is required.

Different types of excipients, as well as several strategies of formulation, are already described to protect bacteriophages from acidic and enzymatic degradations in the stomach and in the beginning of the small intestine.

For instance, WO2006/047872 discloses that whey protein protects phages during freezing and cold storage.

Fatty acids, wax, sugar, cellulose- or sugar-based material are suggested among potential encapsulants. The bacteriophage can be stabilized on a matrix, which can comprise skim milk powder, soya protein, gelatin, peptone, trehalose, mannitol, sugar, sugar alcohol, chitin. The bacteriophage can be, alternatively, embedded in a solution with protein, chitin, cellulose, trehalose... then dried.

US7601347 discloses Eudragit^{®} L100 and sucrose in a dried pharma composition comprising phages. Spray drying of phages with Eudragit^{®} S 100 and sucrose resulted, however, into detrimental reduction in activity.

Khanal et al., 2020, Int. J. of pharmaceutics, discloses spray drying of phages with lactose and L-leucine, followed by tableting (Avicel^{®}, starch,...).

Chang et al. 2017, Eur. J. Pharma and Biopharma, discloses Lactose or trehalose with leucine as excipients during spray drying. Lactose is superior.

Vinner et al., 2019, Pharmaceuticals, discloses spray drying with Eudragit^{®} S100 and trehalose. Trehalose protects Felix O1 phage during spray drying. However, all these formulations lose efficacity after acidic treatment to mimic gastric conditions. Acid stability is speculated to be better with direct compression, further to spray drying. The inlet temperatures range from 100°C to 180°C.

However, to the exception of spray drying or drying on fluidized bed, the drying and encapsulation methods are quite expensive and difficult to scale-up, which is needed to produce enough quantities of stable oral dosage forms to deliver bacteriophages.

### Brief summary of the invention

A first aspect of the present invention is a process for the production of a dry composition comprising a phage comprising the steps of:
- selecting a phage and formulating it in an aqueous solution;
- preparing an aqueous solution comprising a polymeric matrix agent;
- preparing an aqueous solution comprising trehalose and an amino acid selected from the group consisting of valine, leucine, isoleucine, phenylalanine and mixtures thereof;
- optionally combining 2 or the three of the said aqueous solutions;
- submitting the said solutions or the said combined solutions to a spray drying step, wherein the temperature of the drying gas at the inlet is lower than 100°C, preferably of about 80°C.

Advantageously, this process is for protecting the phage towards stomachal acidity.

Preferably, the matrix agent is selected for allowing an intestinal release, preferably a colonic release.

Preferably, the matrix agent is selected from the group consisting of alginate, chitosan, pectin, acrylate and cellulose derivatives, being preferably an acrylate, more preferably a copolymer of methacrylic acid and of a methyl or ethyl methacrylate, still more preferably selected from the group consisting of methacrylic acid - ethyl methacrylate copolymer, methacrylic acid - methyl methacrylate copolymer and Poly(methyl acrylate - methyl methacrylate - methacrylic acid copolymer, more preferably wherein the acrylic acid: methyl or ethyl methacrylate weight ratio ranges between 2:1 and 1:10, preferably between 1:1 and 1:5 or between 1:2 and 1:3.

Advantageously, the matrix agent is an acrylate and further comprises a plasticizer.

Preferably, in this process, at least 10% of trehalose is supplied (weight trehalose : dry weight of the composition).

Preferably, in this process, at least 5% of amino acid, selected from the group consisting of valine, leucine, isoleucine, phenylalanine and mixtures thereof, is supplied (weight amino acid(s) : dry weight of the composition).

Preferably, in this process, at least 60% of the polymeric matrix agent is supplied (weight polymeric matrix agent: dry weight of the composition) and preferably less than 75% of the polymeric matrix agent is supplied (weight polymeric matrix agent: dry weight of the composition).

Preferably, in this process, the composition has a dry weight above 90%, preferably of above 95% (dry wight of the composition : total weight of the composition).

A related aspect of the present invention is an edible pharmaceutical composition comprising the protected phage and obtainable by the process described above.

Another related aspect of the present invention is an edible pharmaceutical composition comprising a matrix consisting essentially of a phage, trehalose, an amino acid selected from the group consisting of valine, leucine, isoleucine, phenylalanine, and mixtures thereof, and a polymeric matrix agent allowing an intestinal release of the said phage.

Preferably, in this edible pharmaceutical composition, the polymeric matrix agent is selected from the group consisting of alginate, chitosan, pectin, acrylate, cellulose derivatives, preferably an acrylate - methacrylate copolymer.

Preferably, the matrix of this edible pharmaceutical composition comprises at least 10% of trehalose (weight trehalose : dry weight of the composition) and/or at least 5% of amino acid (weight amino acid(s) : dry weight of the composition) and/or least 60% of polymeric matrix agent (weight polymeric matrix: dry weight of the composition).

Preferably this edible pharmaceutical composition has a dry weight above 90%, preferably above 95% (dry wight of the composition : total weight of the composition), or the matrix of this edible pharmaceutical composition has a dry weight above 90% (dry wight of the matrix : total weight of the matrix).

Advantageously, this edible pharmaceutical composition comprises more than 10⁷ viable phage per gram (PFU/g).

Another related aspect of the present invention is a pharmaceutically-compatible capsule comprising the edible pharmaceutical composition described here above, such as a pharmaceutically-compatible tablet further comprising a (pharmaceutically-acceptable and/or edible) carrier and/or an excipient, being preferably mannitol and/or cellulose derivatives.

### Brief description of the Drawings

Figure 1 is the stability (spray drying, dark line and pH 2, grey line) of LUZ-19 in function of the relative amount of Eudragit.
Figure 2 is scanning electron microscopy of encapsulated LUZ19.

### Detailed description of an embodiment of the invention

Stomach-resistant capsules are well-known and advantageously allow oral administration of acidic-sensitive substances and/or a direct intestinal release of substances.

On the other hand, as it will be shown below, the resistance to desiccation of the phage composition is impaired by the incorporation of the matrix agent. Conversely, the stability of the powder upon storage is also lower when a matrix agent has been added.

Hence these two features (availability of robust acidity-resistance capsule and difficulties in the formulation/stability with a matrix agent) discourage one to search for a powder composition being inherently resistant to the stomachal acidity, due to the formulation with a matrix agent.

Against this prejudice, the inventors have identified a method of drying phages in a composition with a matrix agent while substantially preserving phage viability. This method is easily up scalable.

Therefore, a first aspect of the present invention is a process for the production of a dry composition comprising a phage comprising the steps of:
- selecting a phage and formulating it in an aqueous solution;
- preparing an aqueous solution comprising a polymeric matrix agent;
- preparing an aqueous solution comprising trehalose and an amino acid selected from the group consisting of valine, leucine, isoleucine, phenylalanine and mixtures thereof;
- submitting the said solutions to a spray drying step, wherein the temperature of the drying gas at the inlet is lower than 100°C, preferably of about 80°C.

This method allows an efficient drying of the phage with limited loss of activity.

The three aqueous solutions can be combined before drying, or can be inserted together in the spray drying.

Preferably, the three solutions are combined, then moved in a tubing, advantageously with a peristaltic pump, finishing in the inlet of the spray dryer.

Advantageously, the temperature of the inlet is lower than 100°C. The inventors have succeeded in obtaining good drying conditions at such a temperature, for instance at 80°C. This better preserves phage viability. However, such low temperatures are perceived as problematic for spray drying, since the parameters have to be adapted, such as the droplet size, which will detrimentally affect the flowability in the drying system, risking hampering the use of spray drying at a large scale.

Advantageously, during this process, specific compounds, preferably the matrix compounds, are chosen for protecting the phage towards stomachal acidity.

Preferably, the matrix agent is selected from the group consisting of alginate, chitosan, pectin, acrylate and cellulose derivatives.

In the context of the present invention, the wording "cellulose derivative", when applied to the matrix agent, preferably refers to cellulose acetate phthalate or to hydroxy propyl methyl cellulose. As it will be shown below, other cellulose derivatives can be used, such as microcrystalline cellulose or methylcellulose, preferably for the tableting and/or for other formulations, subsequent to the drying step.

A preferred matrix agent (used in this process, and in the corresponding edible pharmaceutical composition; see below) is an acrylate (on an acrylate base), and more preferably is a copolymer of methacrylic acid and of a methyl or ethyl methacrylate.

Some of these copolymers are commercialized under Eudragit^{®} trademark and are known to allow an intestinal release of substances. However, on the other hand, the incorporation of large amounts of such a compound in the phage formulation affects the phage viability and dilute the phage concentration, as well as the concentration of the other excipients, including of molecule to protect the phages during the drying step. The incorporation of such matrix agent, at a high concentration, thus represents an additional challenge.

Advantageously, the copolymer of methacrylic acid and of a methyl or ethyl methacrylate is selected from the group consisting of (i) methacrylic acid - ethyl methacrylate copolymer, (ii) methacrylic acid - methyl methacrylate copolymer and (iii) Poly(methyl acrylate - methyl methacrylate - methacrylic acid copolymer.

Preferably, or in addition, when the matrix agent is an acrylate, a plasticizer is further incorporated, preferably in a weight amount plasticizer : acrylate between 1:5 and 1:20, preferably between 1:8 and 1:10, such as about 1:10. A convenient plasticizer is tributyl citrate or triacetin, or triacetin with poly ethylene glycol (PEG; such as PEG 200 or PEG 6000), propylene glycol, diethyl phthalate, and/or oleic acid. Dibutyl phthalate can also be used as a plasticizer.

The inventors have found that the plasticizer reduces the detrimental agglomeration during spray drying.

Preferably, or in addition, when the matrix agent is an acrylate, the acrylic acid: methyl or ethyl methacrylate weight ratio ranges between 2:1 and 1:10, preferably between 1:1 and 1:5 or between 1:2 and 1:3.

Advantageously, the matrix agent is selected for allowing an intestinal release, preferably a colonic release.

This preserves the phage viability and/or allows a direct release at the target site.

Preferably, in this process, at least 10% of trehalose is supplied (weight trehalose : dry weight of the composition).

Preferably, in this process, at least 5% of amino acid, selected from the group consisting of valine, leucine, isoleucine, phenylalanine and mixtures thereof, is supplied (weight amino acid(s) : dry weight of the composition).

In the context of the present invention, the wording "amnio acid" preferably refers to isolated amino acids and/or to monomers (i.e. bearing a free amino and a free carboxyl group; amino and carboxyl groups not engaged in peptide bonds). Isoleucine is the preferred amino acid.

Preferably, in this process, at least 60% of the polymeric matrix agent is supplied (weight polymeric matrix agent: dry weight of the composition) and preferably less than 75% of the polymeric matrix agent is supplied (weight polymeric matrix agent: dry weight of the composition).

The inventors have identified optimal relative amounts of the dry components. Indeed, these components are essential and should be present in high amount. However, the incorporation of more of one essential component means a reduction of another essential component, meaning a reduced flexibility to select the desired amounts. In other words, the inventors have identified a range of optimal efficiency during spray drying.

Preferably, this process is carried out until the composition has a dry weight above 90%, preferably of above 95% (dry wight of the composition : total weight of the composition).

The inventors have found that these high values are important for a long-term preservation of phage viability.

Another related aspect of the present invention is an edible pharmaceutical composition comprising the protected phage. Preferably such pharmaceutical composition is advantageously obtainable by the process described above.

Another related aspect of the present invention is thus an edible pharmaceutical composition comprising a matrix consisting essentially of a phage, trehalose, an amino acid selected from the group consisting of valine, leucine, isoleucine, phenylalanine, and mixtures thereof, and a polymeric matrix agent allowing an intestinal release of the said phage.

Preferably, this polymeric matrix agent is selected from the group consisting of alginate, chitosan, pectin, acrylate, cellulose derivatives, preferably an acrylate - methacrylate copolymer (see in the process above the description of the preferred acrylate present in this edible pharmaceutical composition).

Advantageously, the matrix comprises at least 10% of trehalose (weight trehalose : dry weight of the composition) and/or at least 5% of amino acid (weight amino acid(s) : dry weight of the composition) and/or least 60% of the polymeric matrix agent (weight polymeric matrix: dry weight of the composition; see in the process above the description of the preferred amino acids present in this edible pharmaceutical composition).

The matrix comprising trehalose, the amino acid, the phage and the matrix agent has preferably a dry weight above 90%, preferably above 95% (dry wight of the matrix : total weight of the matrix).

Preferably, this edible pharmaceutical composition has a dry weight above 90%, preferably above 95% (dry wight of the composition : total weight of the composition).

Preferably, this edible pharmaceutical composition comprises more than 10⁷ viable phage per gram (PFU/g).

Preferably, this edible pharmaceutical composition is in the form of a tablet or encapsulated. Advantageously, this edible pharmaceutical composition, when encapsulated, is not in an acid-protected capsule.

The tableting is advantageously achieved with mannitol and cellulose derivatives, preferably microcrystalline cellulose and/or methylcellulose (preferably internally crosslinked methyl cellulose); weight of cellulose derivative and mannitol :total weight of the tablet being comprised between 80:20 and 98:2, preferably between 90:10 and 96:4. In other words, in this tablet, the spray dried powder comprising the phage, the matrix agent, trehalose and the amino acid represents between 2 and 20 weight percent of the tablet.

Other characteristics and advantages of the present invention will be derived from the non-limitative following description, and by making reference to the drawings and the examples.

### Examples

### Materials

The lytic P. aeruginosa bacteriophage, a podovirus named LUZ19 with propagation strain PAO1K (2.0 E+11 pfu/mL stock titer), was used as a model of phage. Bacteriophage LUZ19 was provided by Professor Rob Lavigne (KU Leuven, Belgium) and propagation strain PAO1K were obtained from the Queen Astrid Military Hospital (Brussels, Belgium). LUZ19 has already been disclosed.

Eudragit^{®} FS 30D, an aqueous dispersion of anionic copolymer based on methyl ACRYLate, methyl methACRYLate, methACRYL-ic acid as well as PlasACRYL^{®} T20, and Aerosil^{®} 200 VV Pharma were purchased from Evonik. PlasACRYL^{®} T20, is a 20% emulsion made of sliding agent and plasticizer. It is composed of water, glycerol monostearate, triethyl citrate and polysorbate 80.

### Bacteriophage stock preparation

Bacteriophage stocks were prepared using the double agar overlay method. Briefly, the equivalency of Optical Density (OD) to colony-forming unit (cfu)/mL was pre-determined for the P. aeruginosa strain PAO1 K. The bacteriophage/bacteria ratios were adjusted for efficient reproduction of P. aeruginosa LUZ19 bacteriophage to obtain a "web-pattern" on square petri dishes. The mix of equal volumes (250 µl) phage and bacteria in 15 ml tube was filled with molten agar (46°C) up to 8 ml and overlaid on the surface of solid agar in the square petri dish. Liquid lysogeny broth (LB broth Miller, Animal Free Powder) with 0.6% (soft layer) and 1.5% (solid layer) agar was used. After incubation at 32 °C for 18 h, the bacteriophages (soft layer) were harvested, centrifuged at 6000 g for 30 min, and further filtered using 0.22 µm membrane filters. Then, the lysate was exposed to high-speed centrifugation at 35,000 g for one hour and a half, and the bacteriophage pellet was resuspended in Dulbecco's Phosphate-Buffered Saline (DPBS) without (w/o) Ca2+ and Mg2+ and stored at 4 °C. The titers for each propagated bacteriophage stock (2.0E+1 1 pfu/ml) were determined by the double agar overlay method.

Temperature stability was assessed by incubating 1 mL of 10⁸ PFU/mL phage samples at different temperatures (25°C, 37°C, 45°C, and 60°C). For pH stability, 100 µL of 10⁹ PFU/mL phage samples were diluted in 900 µL of PBS at different pH (2, 4, 6, 8, 10, and 12). All samples were incubated for 1 h and subsequently titrated in bio-logical triplicate.

### Spray-drying

The bi-fluid nozzle was used with formulations containing different D-(+)-trehalose dihydrate with L-Isoleucine (63,3 %w/w and 36,7 %w/w, respectively) and Eudragit^{®} FS-30D with PlasACRYL^{®} T20 (90,9 %w/w and 9,1 %w/w, respectively) weight ratios (Table 1). These formulations were spray-dried as 5% (w/v) aqueous solutions with 1% v/v of bacteriophages separately using a Büchi Mini Spray-dryer B-290 with a Büchi dehumidifier B296. The experiments were performed in the open mode, coupled with a 0.7 mm two-fluid nozzle, and using air as the drying gas in a co-current mode. The formulation was fed at a constant feed rate of 2 mL/min, an aspiration rate of 35 m³/h, a Tᵢₙ (temperature of the drying gas at the top of the column) of 80°C and a spray gas flow of 819 L/h. After processing, each powder was stored in sealed type 1 clear pharmaceutical grade glass amber vials. Stoppers used were ultra-pure chlorobutyl-isoprene rubber stoppers, sealed to the vial with aluminum caps.

**Table 1. Formulations of the microspheres. Initial mixture Trehalose/Isoleucine - 63,3/36,7 %w/w; initial mixture Eudragit^{®} FS/PlasACRYL^{®} - 90,9/9,1 %w/w.**

| **Formulations** | **% w/w D-(+)- Trehalose and Isoleucine** | **% w/w Eudragit^{®} FS-30D with PlasACRYL^{®} T20** |
|---|---|---|
| | | |
| F1 | 100 | 0 |
| F2 | 50 | 50 |
| F3 | 25 | 75 |
| F4 | 20 | 80 |
| F5 | 15 | 85 |
| F6 | 10 | 90 |
| F7 | 5 | 95 |
| F8 | 0 | 100 |

### Bacteriophage stability titration

For each formulation, the stability of bacteriophages was assessed under acidic (pH 2.0) and neutral conditions (pH 7.4) (n=3). 10 mL of HCl 0.37% were introduced in the vials containing an exact amount of powder. The particles were in contact with the acidic media for 2 hours before being filtered under vacuum with Durapore^{®} 0.22 µm PVDF membrane. Then, they were placed in vials to be dispersed in 10 mL of DPBS (pH=7.4) w/o Ca²⁺ and Mg²⁺. Samples (n=3) were also immediately reconstituted in 10 mL of DPBS as positive control.

The spot testing on agar overlay technique was used to determine the number of viable bacteriophages (PFU/mL) in the powder samples. Briefly, ten-fold serial dilutions of samples were made in 96-well microplates by adding 20 µL samples to 180 µL DPBS. All sample dilutions were carried out in triplicate. Bacterial lawns were made by mixing 300 µL of OD 0.3 host bacterial suspension with up to 8 mL of molten soft agar (0.7% LB, 46 °C) and overlaid onto pre-prepared square petri dishes with 1.5% LB agar. A volume of 2 µL diluted bacteriophage samples were spotted onto the soft agar surface, and test plates were air-dried in a biosafety cabinet and incubated up-side down at 32 °C for 18 h. The number of pfu that were contained in 2 µL was ex-pressed in pfu/ml. Every test plate included a standard bacteriophage sample with pre-defined titer (pfu/ml) as a control of the titration process. Finally, the titer was converted from pfu/mL to pfu/mg, considering the initial mass of powder that was present in the vial.

For each step, titration triplicates were done to ensure uniformity of the batch and biological triplicates were done for the analytical method.

Yields of production were calculated based on the weight of powder that was collected after spray-drying in the spray dryer collection vessel and are expressed as a percentage of the weight of dry materials introduced into the feed solution (w/w%).

Scanning Electron Microscopy (SEM), using an SU8020 SEM-FEG microscope (Hitachi), was performed to evaluate the morphology of the dried particles. The samples were prepared by sticking the powder to a circular shelf using an adhesive carbon tab before coating with 2.5 nm of Pt/Pd using a Leica ACE600 metallizer. The images were acquired at a voltage of 5 kV using the secondary electron detector SE (L) located in the chamber. The higher magnification images were acquired at a voltage of 2 kV, using the SE (U) detector located in the column. The magnifications used were 1000, 5000, 30000, and 50000x.

The amount of residual water was assessed by thermogravimetric analysis (TGA), using a TA instruments Q500 apparatus and Universal Analysis 2000 (version 4.4A) software (TA Instruments, Belgium). About 10 mg of powder were placed on the platinum cup and were subjected to a range of temperatures from 30 to 200 °C with a heating rate of 10 °C/min. The remaining moisture content (RMC) was defined as the weight loss between the powders at 30 °C and 120 °C.

Differential scanning calorimetry (DSC) analysis was carried out under nitrogen purge using a DSC Q2000 instrument and Universal Analysis 2000 (version 4.4A) software (TA Instruments, Belgium) to evaluate the glass transition temperature (Tg). Powder samples (2-4 mg) were placed in a Tzero hermetic aluminum pan, sealed, and heated over the temperature range of -50 to 150°C with a heating rate of 10 °C/min.

The powders were analyzed by powder X-ray diffraction (PXRD) using an X-ray diffractometer (D8 Advance Eco Bruker^{®}), equipped with a one-dimensional silicon detector (LynxEye, Bruker AXS) and using Cu-Ka radiation (1.54 Å; 40 kV × 25 mA). Data were collected over the angular range of 4-40° 2θ, with a step size of 0.02° and a dwell time of 2 s. The percentage of amorphous form was calculated as 100% minus the crystalline phase content in the powder, as determined using the surface area ratio method.

The size distribution of the particles was determined by laser-diffraction, using a Malvern Mastersizer^{®} 3000 equipped with an Aero S dry powder disperser unit (Malvern Instruments Ltd).

Samples of dried powder (n = 3) were placed in the hopper of the disperser. During the measurement, 75% vibration was used and a pressure of 4 bar was achieved.

Particle density (ρP) was measured using a helium gas pycnometer Ultrapyc 5000 equipment (Anton Paar), according to the procedure described in the European Pharmacopeia Ph.Eur.10.0,20242(01/2010). Approximately 10 grams of the samples were weighed (triplicate) and transferred to the 10 cm³ sample holder. A pressure of 10 psi was used at a temperature of 20 °C, with true density calculated in g/cm³. The results were obtained from fifteen measurements of volume and density. Loose bulk density (ρL) was measured by using 10 mL plastic graduated cylinder. After passing through a 0.5 mm sieve, the powder is poured into a 10 ml stemmed glass, avoiding any shaking. According to the European Pharmacopeia, Ph.Eur.10.0,20934 (04/2019), the loose bulk density considers the density of the powder particles but also the interparticle void volume. It is given by the ratio between the mass of powder in the stemmed glass and the volume it occupies there. It is expressed in g/ml, g/cm3, kg/m3. Tapped bulk density (ρT) was measured after 1250 mechanically tapping of a graduated cylinder of 10 ml which containing the powder sample. The device used is a Stampvolumeter STAV2003 and has a tap height of 3 mm. The value of 1250 taps was chosen because it corresponds to the number of taps necessary to reach a constant volume of powder. Porosity (ε) was calculated by using the relationship between tapped bulk ρT and ρp. Carr index (CI) was used for representing flowability of powders as described in the European Pharmacopeia Ph.Eur.10.0,20936 (01/2010). Cl was calculated using the tapped bulk density (ρT) and the loose bulk density (ρL). Cl is classified as: <10 very good, 11-15 good, 16-20 fair, 21-25 passable, 26-31 bad and > 32 very bad. Haussner ratio (HR) was used for representing cohesiveness of powders. HR was calculated by taking ratio of ρT and ρL. HR is classified as: <1.19 low, 1.19-1.34 intermediate, and > 1.34 high. To consider having a good flowability, the Cl must be lower than 15% and the HR must be lower than 1,18.

The mixing was made gradually. The spray-dry powder comprising the bacterio-phages was previously mixed to colloidal silicon dioxide. Then the so-called "sandwich" mode of incorporation was applied. That is, in a glass container where the total volume of powder represents 40-60%, half of the microcrystalline cellulose and mannitol was deposited then the premix with croscarmellose and hydroxypropyl cellulose then the other half of microcrystalline cellulose and mannitol. The powder was mixed for 15 minutes with a Turbula^{®} Mixer (WAB) at 33 rpm. Magnesium stearate was added at the end of the process for 1 minute.

**Table 2; Detailed mix for tableting**

| **Excipients/Formulations** | **% w/w** |
|---|---|
| Spray-dried powder | 3.00 |
| Microcrystalline Cellulose | 55.48 |
| Mannitol | 36.39 |
| Colloidal silicon dioxide | 0.10 |
| Hydroxypropyl Cellulose | 2.42 |
| Croscarmellose Sodium | 1.61 |
| Magnesium stearate | 1.00 |
| TOTAL | 100.00 |

The uniformity of capsules has been assessed as described in the European Pharmacopoeia: Ph.Eur.10.0,20940(04/2017). Twenty capsules are taken at random and weighed.

Tablets were prepared by compression. The mixtures were automatically fed into the die of an instrumented single-punch tableting machine (Korch) to produce tablets containing encapsulated bacteriophages using 7 mm concave-faced punches and dies.

Mass uniformity of tablets was tested according to the European Pharmacopeia Ph.eur.10.0,20940(04/2017). 20 units taken at random were weighed and the mean mass and standard deviation were determined. The resistance to crushing of tablets was evaluated according to the European Pharmacopeia Ph.Eur.10.0,20908(01/2008) with a hardness tester (Computest, Kreamer Gmbh, EL Ektronik) on 10 tablets. Friability of tablets Ph.Eur.10.0,20907501/2010) was carried out on 20 tablets by performing 100 rotations of the drum at 20 rpm. The friability was expressed as the percentage of mass loss. The authorized limit loss was 1% w/w. Disintegration test was evaluated according to Ph.Eur.10.0,20901 (01/2022). The apparatus consists of a basket containing 6 tubes (1 tablet per tube) open at both ends containing a wire mesh (2 mm mesh) at the lower part. The basket was immersed in a one litter cylindrical container containing 0.1 N HCl. The temperature was set at 37±1°C.

To know the release profile of formulated LUZ19 bacteriophages in the colon, a modified Simulator of Human Intestinal Microbial Ecosystem (SHIME), an *in vitro* model of the gastro-intestinal tract, was used. For this purpose, the release profile of the formulation F4 was assessed, in triplicate, with three bioreactors. These fermenters were placed on magnetic stirrers (to mimic the dynamic of the gastro-intestinal tract) and maintained at a temperature of 37°C. They were filled with 140ml of a broth called feed (containing pectin, xylan, glucose, potato flour, arabinogalactan, extract of yeast, peptone, mucin, cystein provided by ProDigest, adjusted at pH=2 (with HCl 37%) and were connected to a synthetic pancreatic juice, PJ, (containing sodium hydrogen carbonate, pancreatin, bile salts (ProDigest). The pH of the reactors was adjusted using pH probes which automatically trigger the distribution of acidic (HCl 0.5M) or basic (NaOH 0.5M) solutions (ProDigest) to maintain a specific pH range and which allow the monitoring of the pH in real time.

Briefly, the formulated bacteriophage (1020 mg at ~2.5E+06 PFU/mg) was discharged in the different fermenters filled with feed (n=3). After an incubation of the bacteriophage in the feed during 2 h, 60 ml of PJ was automatically added in each the reactor during 15 min (which gradually conducted the solution to a pH=7). After the entire PJ distribution, the pH of the bioreactors was increased to 7,4. Samples were withdrawn after reaching pH=5, pH=6, pH=7 and pH=7.4. Once pH=7.4 was reached, three other samples were withdrawn after 45 min, 120 min, 240 min and 300 min. All samples were filtrated using 0,2µm syringe filters (ref.514-0073, VWR) and then tenfold serially diluted in PBS (n=3) for the bacteriophage's titration. Drops of 2 µl on the different dilutions were plated on enriched LB agar Petri dishes (with 1mM MgSO₄ and 1 mM CaCl₂) on a soft-agar Pseudomonas overlay and put at 37°C for 12 h. After incubation, the plaques of lysis were counted to evaluate the concentration of the delivered bacteriophages in the SHIME model.

Stability testing was performed according to the ICH harmonized guidelines. Stability testing was conducted on the bacteriophage substances packaged in sealed vials for3 months and tests were performed after 1 day, after 1, 3 months at 4 ± 2 ∘C: 25 ± 2 ∘C and 60% RH; 30 ± 2 ∘C and 65% RH; and 40 ± 2 ∘C and 75% RH in climatic chambers (Weiss).

Statistical analysis was carried out using GraphPad^{™} version 9 (USA). Two-sample t-tests were performed (n = 3) with reporting of p < 0.05 as statistically significant. Where multiple tests were done, the value of alpha was adjusted using the Bonferroni correction. Error bars represent a single standard deviation for the mean values of the replicates.

### Influence of the temperature and pH on bacteriophage activity

LUZ19 remained stable (<1 log PFU/mL reduction) for one hour, regardless of the temperature (25°C, 37°C, 45°C and 60°C). In contrast, the stability of LUZ19 was strongly influenced by the pH. Indeed, LUZ19 were not detectable at pH 2 and pH 12. Moreover, regarding the reference, its loss of activity reached 1.5 log10 at pH 4.0 and 0.6 log10 at pH 10. LUZ19 remained stable at pH 6.0 with no significative loss. These results clearly showed the importance of protecting the bacteriophage model LUZ19 from the acidic environment of the stomach and to promote its release after the pylorus.

LUZ19 was spray-dried at a phage titer 2.0E+09 pfu/mL (2.0 E+11 pfu/mL for the stock titer) with different percentages of D-(+)-trehalose dihydrate and L-lsoleucine (63.3 %w/w and 36.7 %w/w, respectively) and Eudragit^{®} FS-30D with PlasACRYL^{®} T20 (90.9 %w/w and 9.1 %w/w respectively) weight ratios (Table 1).

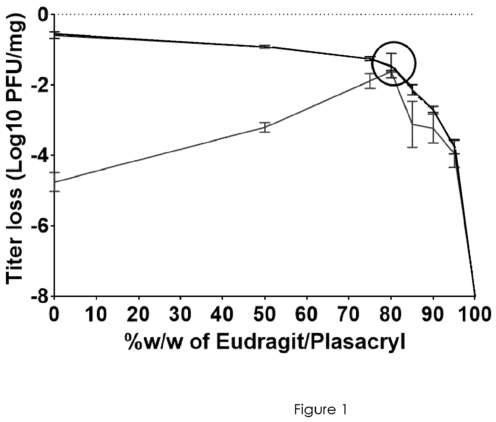

The effect of the different ratios of these excipients on phage activity following the spray-drying process was evaluated. The spray-dried phage-containing powders were exposed to acidic conditions at pH 2.0 for 2 h and the resulting phage activity was evaluated (Figure 1).

The results obtained after spray-drying for the formulation containing only D(+) trehalose and L-lsoleucine without Eudragit^{®} and PlasACRYL^{®} T20 (F1) show a marginal loss of activity of -0.59 ± 0.09 log 10 (Figure 1, dark line).

However, this formulation did not protect bacteriophages from acid conditions which results in a loss of -4.76 log10 PFU/mg (figure 1, grey line).

When the ratio of Eudragit^{®} FS30D /PlasACRYL^{®} T20 is increased, the phage was less stable after spray-drying. The inventors thus concluded that a minimal amount of D-(+)-Trehalose was needed to protect bacteriophages from desiccation. In contrast, its stability increased upon contact with acidic medium due to an increase in the total percentage of Eudragit^{®} FS.

A ratio Trehalose/L-Isoleucine: Eudragit^{®} FS30D/PlasACRYL^{®} T20 of 20:80 (F4) has shown a loss of activity of-1.46 log10 PFU/mg after process and, interestingly, no additional loss of activity after 2 hours at pH 2.0. Then, it was surprisingly observed that an increase of the percentage Eudragit^{®} FS/PlasACRYL^{™} in F5, F6 and F7 was deleterious for the bacteriophage after spray-drying. Indeed, it could be seen that the loss of activity after 2 hours at pH 2.0 was due to the initial loss of activity after process. Using 100%w/w of Eudragit^{®} FS30D/PlasACRYL^{®} T20 in F8, LUZ19 was totally inactivated, already after spray-drying.

The inventors conclude that, even if the Eudragit^{®} FS30D allows protecting LUZ-19 from acid medium, a minimum of trehalose/amino acid was needed to protect the bacteriophages from desiccation stresses.

### Characterization of Eudragit^{®} FS-based microparticles

The yield of atomization decreased from 90% to 45% w/w when the percentage of Eudragit^{®} FS/PlasACRYL^{®} T20 increased (Table 1). For instance, F4 which contained 80% w/w of Eudragit^{®} FS/PlasACRYL^{®} T20 has shown a yield of 70% w/w.

The moisture content of spray-dried powders ranged between 1 and 4% (w/w). It was shown that, the higher the Eudragit^{®}/PlasACRYL^{®} T20 content, the drier the powder. As previously observed, a decrease of the moisture content increased the electrostatic charges which reduced the yield of the process.

The Tg was found to be inversely correlated to the RMC. Indeed, it is well known that the moisture content is exponentially correlated to the Tg (higher the moisture content is, lower is the Tg), as described in the Gordon-Taylor equation. It is important to consider these parameters in view of stability of the powder over time. Indeed, bacteriophage viability in a powder is closely associated with the temperature gap between the storage temperature (Ts) and the Tg. On the other hand, when the level of Eudragit^{®} FS/PlasACRYL^{®} T20 increased, the percentage of amorphous structure in the whole spray-dried powder increased to be ranged from 44% (0% of Eudragit^{®} FS/Plasacryl^{™} T20 in F1) to 76.8% (100% of Eudragit^{®} FS Plosocryl^{™} T20 in F8). Due to the high rate of crystallization of L-Isoleucine, when its amount decreased from F1 to F8, the percentage of amorphous form of the powder increased.

The different spray-dried powders were characterized by similar particle size distributions (Table 3). The Dv50 was ranged between 2.5 and 3.5 µm.

**Table 3. Particle size analysis of different powder containing LUZ19 encapsulated.**

| **Formulation** | | **Dv10 (µm)** | **Dv50(µm)** | **Dv90(µm)** |
|---|---|---|---|---|
| F1 | Average | 0,87 | 2,54 | 6,27 |
| | Standard deviation | 0,00 | 0,04 | 0,22 |
| F4 | Average | 1,19 | 2,97 | 6,64 |
| | Standard deviation | 0,00 | 0,02 | 0,09 |

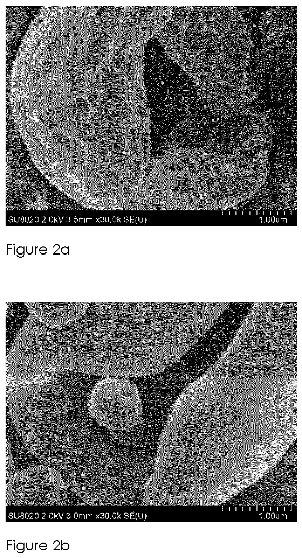

Particles containing only Trehalose and L-Isoleucine (F1) were characterized by a rough surface (Figure 2a). For particles containing 80% w/w of Eudragit^{®} FS30D/PlasACRYL^{®} T20 (F4), the surface was clearly smoother (Figure 2b). Being in the form of a latex dispersion composed soft nanoparticles which may fuse during the atomization, an increase in the percentage of Eudragit^{®} FS produced a smoother aspect of the phages-loaded microparticles.

### Characterization of the flowability

The flowability of F1 and F4 was characterized to evaluate the influence of Eudragit^{®} FS as F1 did not contain the colonic-targeting polymer. As it can be seen in Table 4, both powders were characterized as very, very poor according to the European Pharmacopeia Ph.Eur.10.0,20936 (01/2010) (Table 4). Nevertheless, the powder containing Eudragit^{®} FS had a better flowability (e.g., lower Carr's index and Hausner ratio). The size and density of the particles being similar, this difference could be due to the smoother surface of these particles (F4).

Table 4. Characteristics regarding flowability of the non-encapsulated (F1) and the optimal encapsulated (F4) powder containing LUZ19 (ρP: particle density; ρL: Loose bulk density; ρT: Tapped bulk density; ρ: Porosity; Cl: Carr's index; HR: Hausner ratio)

| **Formulation** | **ρ*P* (g/cm3)** | **ρ*L* (g/cm3)** | ***ρT* (g/cm3)** | ***ε*(%)** | **CI (%)** | **HR** |
|---|---|---|---|---|---|---|
| F1 | 1,3891 | 0,1093 | 0,3080 | 77,82 | 64,51 | 2,82 |
| F4 | 1,3664 | 0,1790 | 0,3016 | 77,93 | 40,65 | 1,68 |

Capsules of 3 different sizes (00, 0 and 1) were filled with F1 and F4 spray-dried powders. A mass uniformity test of the capsules was carried out to ensure the homogeneous filling of the capsules. The capsules with F4 were all compliant.

The powder only composed by Trehalose-Isoleucine (F1) was more hygroscopic and its flow in the capsules was less fluid than the powder containing Eudragit^{®} FS and PlasAcryl^{®} (F4). Therefore, the use of gastro-resistant capsules which could be filled with Eudragit^{®} FS-free powder (F1) seemed to be useless. Indeed, powders (F4) containing Eudragit^{®} FS have already shown their ability to protect the phage from acidity. Standard capsules, which is commonly used in compounding pharmacy, are suitable. Since the use of phages is dedicated to the treatment of specific pathogenic bacteria, the use of standard capsules facilities the implementation of individual medicine. Such capsules may also be opened to disperse the colon-targeted phage-loaded powder in water for patients with swallowing issues.

### Characterization of the mix for tableting

In addition to their use in capsules, the developed spray-dried powders may also be incorporated in tablets. Such dosage form cannot be produced in hospital or compounding pharmacy but minitablets may be used for personalized medicine. Moreover, it was shown that our spray-dried phage-loaded powders were characterized by poor flowability. Therefore, carriers had to be added to promote the proper flowability of the whole mixture (Table 2). As it was expected, significant improvement was observed (Table 6). For instance, the flowability of the mixture containing F4 increased from very poor to passable according to the European Pharmacopeia Ph.Eur.10.0,20936 (01/2010).

**Table 6 . Characteristics regarding flowability of the mix containing encapsulated powder containing LUZ19 (ρP: particle density; ρL: Loose bulk density; ρT: Tapped bulk density; ε: Porosity; Cl: Carr's index; HR: Hausner ratio)**

| **Formulation** | **ρ*P* (g/cm3)** | **ρ*L* (g/cm3)** | **ρ*T* (g/cm3)** | ***ε*(%)** | **CI (%)** | **HR** |
|---|---|---|---|---|---|---|
| F4+mix | 1.5407 | 0,4336 | 0,5781 | 62,48 | 25 | 1,33 |

The physical properties of the mixtures made of spray-dried microparticles (F4) and excipient dedicated for compression were suitable to properly produce robust tablets.

### Characterization of tablets

The average mass of the tablets was found to be 155 ± 2 mg. As the pharmacopoeia recommendations states that the individual mass of not more than 2 of the 20 tablets deviated from the average mass by a higher percentage of 10%, and the mass of any unit did not deviate by more than double that percentage, it could be concluded that the mass uniformity of the tablets was conform. In addition, the loss of mass after the friability test was 0.9 % w/w which was under the recommended limit loss of 1%. Their hardness was found to be 112 ± 8.4 N. Their disintegration time was evaluated at 2 min 34 sec in DPBS and 5min 6 sec in HCl 0.1N which was lower than the conventional 15 minutes that are allowed from standard tablets.

F4 powder was evaluated in terms of acid stability after formation of tablets. The results showed no significant difference before and after tableting.

Activity release test of LUZ19 from powder with Simulator of the Human Intestinal Microbial Ecosystem (SHIME)

At pH 5 and 6, no bacteriophage activity was found. At pH 7.4), the release of the LUZ19 bacteriophage started and was sustained over 5 hours. Before pH 7.0, the enteric polymer was insoluble and preserved the release of the phage. At pH 7.4, the polymer stared to dissolved which initiated the release of LUZ19. The release was sustained for more than 5 hours due to the progressive erosion of the dosage form. Such data showed that the spray-dried system developed by the inventors was a solid dispersion in which the bacteriophage was entrapped in a matrix based on the enteric polymer.

This profile of dissolution is ideal for disinfecting or decolonizing all along the ileum, caecum, and proximal colon according to the pH and transit time. Thus, the bacteriophages could amplify all along.

### Stability tests of the powder

A storage stability study at different ICH conditions was conducted with the most promising formulation in terms of acid stability (F4).

At +5°C, no significative difference in activity was observed over time and between direct dissolution of powder in DPBS at pH 7.4 and 2h in acid as well as after dissolution in DPBS at pH 7.4. Therefore, it was concluded that the encapsulated LUZ19 produced was stable at +5°C over 3 months.

Moreover, no deterioration of the acid protection over time at this temperature was observed. However, at higher temperature and humidity, the activity decreased after 1 month of storage before being stabilized between 1 and 3 months. As there was no significative difference between neutral and acid conditions, it was hypothesized that there was no deterioration of the enteric polymer. The inventors attribute the loss of activity to the reduced content of D-(+)-Trehalose and L-lsoleucine compared to the formulation without Eudragit^{®} FS and PlasAcryl.

It should be understood that the present invention is not limited to the described embodiments and that variations can be applied without going outside of the scope of the claims.

## Claims

1. A process for the production of a dry composition comprising a phage comprising the steps of:
- selecting a phage and formulating it in an aqueous solution;
- preparing an aqueous solution comprising a polymeric matrix agent;
- preparing an aqueous solution comprising trehalose and an amino acid selected from the group consisting of valine, leucine, isoleucine, phenylalanine and mixtures thereof;
- optionally combining 2 or the three of the said aqueous solutions;
- submitting the said solutions or the said combined solutions to a spray drying step, wherein the temperature of the drying gas at the inlet is lower than 100°C, preferably of about 80°C.

2. The process of claim 1, being further for protecting the phage towards stomachal acidity and/or wherein the matrix agent is selected for allowing an intestinal release, preferably a colonic release.

3. The process according to any one of the preceding claims, wherein the matrix agent is selected from the group consisting of alginate, chitosan, pectin, acrylate and cellulose derivatives, being preferably an acrylate, being more preferably a copolymer of methacrylic acid and of a methyl or ethyl methacrylate, still more preferably selected from the group consisting of methacrylic acid - ethyl methacrylate copolymer, methacrylic acid - methyl methacrylate copolymer and Poly(methyl acrylate - methyl methacrylate - methacrylic acid copolymer

4. The process of claim 3, wherein the matrix agent is an acrylate further comprising a plasticizer.

5. The process of claims 3 or 4, wherein the acrylic acid: methyl or ethyl methacrylate weight ratio ranges between 2:1 and 1:10, preferably between 1:1 and 1:5 or between 1:2 and 1:3.

6. The process according to any one of the preceding claims, wherein at least 10% of trehalose is supplied (weight trehalose : dry weight of the composition).

7. The process according to any one of the preceding claims, wherein at least 5% of amino acid, selected from the group consisting of valine, leucine, isoleucine, phenylalanine and mixtures thereof, is supplied (weight amino acid(s) : dry weight of the composition).

8. The process according to any one of the preceding claims, wherein at least 60% of the polymeric matrix agent is supplied (weight polymeric matrix agent: dry weight of the composition) and preferably less than 75% of the polymeric matrix agent is supplied (weight polymeric matrix agent: dry weight of the composition).

9. The process according to any one of the preceding claims, wherein the composition has a dry weight above 90%, preferably of above 95% (dry wight of the composition : total weight of the composition).

10. An edible pharmaceutical composition comprising the protected phage and obtainable by the process of claim 9.

11. An edible pharmaceutical composition comprising a matrix consisting essentially of a phage, trehalose, an amino acid selected from the group consisting of valine, leucine, isoleucine, phenylalanine, and mixtures thereof, and a polymeric matrix agent allowing an intestinal release of the said phage, preferably the said edible pharmaceutical composition having a dry weight above 90%, preferably above 95% (dry wight of the composition : total weight of the composition), or wherein the matrix has a dry weight above 90% (dry wight of the matrix : total weight of the matrix).

12. The edible pharmaceutical composition of claim 11, wherein the polymeric matrix agent is selected from the group consisting of alginate, chitosan, pectin, acrylate, cellulose derivatives, preferably an acrylate - methacrylate copolymer.

13. The edible pharmaceutical composition of claim 11 or 12, wherein the matrix comprises at least 10% of trehalose (weight trehalose : dry weight of the composition) and/or at least 5% of amino acid (weight amino acid(s) : dry weight of the composition) and/or least 60% of polymeric matrix agent (weight polymeric matrix: dry weight of the composition).

14. The edible pharmaceutical composition according to any one of the preceding claims 10 to 13 comprising more than 10⁷ active phage per gram (PFU/g).

15. A pharmaceutically-compatible capsule comprising the edible pharmaceutical composition according to any one of the preceding claims 10 to 14, or a pharmaceutically-compatible tablet comprising the edible pharmaceutical composition according to any one of the preceding claims 10 to 14 and a carrier and/or and excipient, the said carrier and/or and excipient comprising preferably mannitol and/or cellulose derivatives.
